(19) Europäisches Patentamt
European Patent Office
Office européen des brevets

(11) Veröffentlichungsnummer: **0 267 537 B1**

(12) **EUROPÄISCHE PATENTSCHRIFT**

(45) Veröffentlichungstag der Patentschrift: **08.01.92**

(21) Anmeldenummer: **87116295.4**

(22) Anmeldetag: **05.11.87**

(51) Int. Cl.⁵: **C07C 45/67**, C07C 45/72, C07C 45/29, C07C 49/573, C07C 49/242, C07C 49/553, C07C 49/203, C07C 49/794

# (54) Verfahren zur Herstellung von Vinylglyoxalderivaten, neue Vinylglyoxalderivate und deren Verwendung.

(30) Priorität: **11.11.86 DE 3638489**

(43) Veröffentlichungstag der Anmeldung:
**18.05.88 Patentblatt 88/20**

(45) Bekanntmachung des Hinweises auf die Patenterteilung:
**08.01.92 Patentblatt 92/02**

(84) Benannte Vertragsstaaten:
**CH DE FR GB IT LI NL**

(56) Entgegenhaltungen:
**FR-A- 1 350 741**

**ANGEW. CHEMICAL, Band 98, Nr. 12, 1986, Seiten 1134-1136, VCH Verlagsgesellschaft mbH; R. KRAMME et al.: "Divinylglyoxal und methylvinylglyoxal"**

(73) Patentinhaber: **BASF Aktiengesellschaft**
**Carl-Bosch-Strasse 38**
**W-6700 Ludwigshafen(DE)**

(72) Erfinder: **Neumann, Peter, Dr.**
**Franz-Schubert-Strasse 1**
**W-6908 Wiesloch(DE)**
Erfinder: **Martin, Hans-Dieter, Prof. Dr.**
**Universitätsstrasse**
**W-4000 Düsseldorf(DE)**
Erfinder: **Kramme, Roland**
**Tannenweg 27**
**W-4401 Hilten(DE)**
Erfinder: **Weimann, Thomas**
**Volmerswerther-Strasse 442**
**W-4000 Düsseldorf(DE)**

**EP 0 267 537 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

SYNTHESIS, April 1980, Seiten 309-310, Georg Thieme Verlag; H. STETTER et al.: "Über die präparative Nutzung der 1,3-Thiazoliumsalz-katalysierten acyloin- und benzoin-bildung; III. Eine neue Methode zur Herstellung von substituierten Enol-trimethylsilyl-ethern des 1,2-Cyclopentandions"

C.R. ACAD. SC. PARIS, t 272, 11. Januar 1971, Seiten 233-235, Serie C; S.-L.-T. THUAN et al.: "L'oxydation sélective des diol- 1,2 diéthyléniques alpha-dicétones diéthyleniques"

JOURNAL OF THE AMERICAN CHEMICAL SOCIETY, Band 97:7, 2. April 1975, Seiten 1900-1905; G.A. RUSSELL et al.: "Aliphatic semidiones. XXX. Alkenyl and cycloalkyl substituted 1,2-semidiones"

CHEMICAL ABSTRACTS, Band 71, 1969, Seiten 307-308, Nr. 21941p, Columbus, Ohio, US; I.G. TISHCHENKO et al.: "Epoxy compounds of beta,beta-dialkyldivinyl ketones and unsaturated beta-methoxy ketones"

## Beschreibung

Die Erfindung betrifft ein Verfahren zur Herstellung von Vinylglyoxalderivaten, neue Vinylglyoxalderivate sowie deren Verwendung als Monomerkomponenten in polymeren Systemen.

Das Methylvinylglyoxal der Formel (I'a)

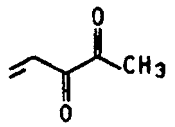

(I'a)

ist als Bestandteil der Dionfraktion bei der troposphärischen Photooxidation von aromatischen Kohlenwasserstoffen postuliert worden, vgl. J. Phys. Chem. 88, 4122 (1984), die Verbindung als solche wurde aber weder isoliert, noch läßt sich aus der Literaturstelle ein Verfahren zu deren Herstellung ableiten. Auch über die Eigenschaften der Verbindung ist in der Literaturstelle nicht ausgesagt.

Das Divinylglyoxal der Formel (I'b)

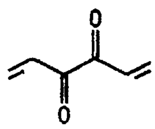

(I'b)

wird in C.R. Acad. Sc. Paris, Serie C, Seiten 233 bis 235 (1971) zwar formelmäßig genannt, es wird auch angegeben, daß die Verbindung in "Spuren" auftreten soll. Eine Charakterisierung dieser Verbindung findet sich in der Druckschrift nicht, ebensowenig eine Arbeitsweise, welche die Herstellung dieser Verbindung in einer Menge gestattet, welche ihre Reindarstellung und Charakterisierung erlaubt. Da in der Druckschrift auch keine experimentellen Angaben über die angebliche Herstellung und eine etwaige Charakterisierung gemacht sind, muß davon ausgegangen werden, daß diese Verbindung rein spekulativ genannt ist, somit der Fachwelt nicht zugänglich gemacht wurde.

Der Erfindung liegt die Aufgabe zugrunde, ein Verfahren zur Herstellung von Vinylglyoxalderivaten der allgemeinen Formel (I)

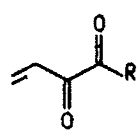

(I)

worin R für einen geradkettigen oder verzweigten Alkyl- oder Alkenylrest oder Cycloalkylrest oder einen gegebenenfalls substituierten Phenylrest steht, zur Verfügung zu stellen.

Diese Aufgabe wird gelöst durch ein Verfahren, das dadurch gekennzeichnet ist, daß man Bicyclo-(2,2,1)-hept-5-en-carbaldehyd der Formel V

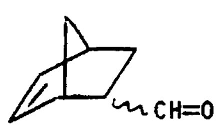

(V)

in Gegenwart eines Katalysators entweder

(a) mit sich selbst unter Bildung einer Verbindung der allgemeinen Formel VII

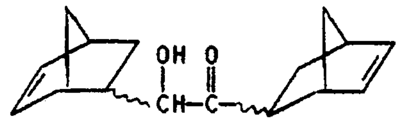

(VII)

reagieren läßt, oder
(b) mit einer Verbindung der allgemeinen Formel II

R—CH=O (II)

worin R die oben angegebenen Bedeutungen besitzt, unter Bildung eines Gemisches der Verbindungen der allgemeinen Formeln VIII (a) und (b)

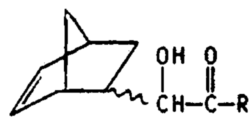

(VIII a)

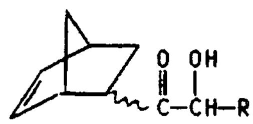

(VIII b)

worin R wie zuvor definiert ist, umsetzt,
die Verbindungen der allgemeinen Formeln VII beziehungsweise VIII (a) und (b) zu den entsprechenden Dicarbonylverbindungen oxidiert und anschließend bei einer Temperatur von 500 bis 700 °C und unter vermindertem Druck, insbesondere einem Druck von weniger als $10^{-1}$ mbar eine Gasphasenpyrolyse durchführt, und gewünschtenfalls die gebildeten Verbindungen der allgemeinen Formel I weiter reinigt.

Nach einer bevorzugten Ausführungsform des erfindungsgemäßen Verfahrens führt man die Gasphasenpyrolyse bei 550 bis 630 °C, insbesondere 580 bis 600 °C, und einem Druck von ungefähr $10^{-2}$ mbar durch.

Nach einer bevorzugten Ausführungsform des erfindungsgemäßen Verfahrens setzt man eine Verbindung der allgemeinen Formel II ein:

R—CH=O (II)

worin R für $C_1$-$C_{20}$-Alkyl, insbesondere $C_1$-$C_4$-Alkyl, $C_2$-$C_{20}$-Alkenyl, insbesondere $C_2$-$C_6$-Alkenyl, $C_3$-$C_7$-Cycloalkyl, oder gegebenenfalls durch Halogen, Cyan, $C_1$-$C_{12}$-Alkyl, insbesondere $C_1$-$C_4$-Alkyl, $C_1$-$C_{12}$-Alkoxy, insbesondere $C_1$-$C_4$-Alkoxy oder $CF_3$-substituiertes Phenyl, steht.

Gegenstand der Erfindung sind auch Verbindungen der allgemeinen Formel (I')

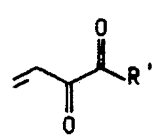

(I')

worin R' für Methyl, Ethyl, n-Propyl, iso-Propyl, n-Butyl, iso-Butyl, Hexyl, Heptyl, Octyl, $C_2$-$C_{20}$-Alkenyl, $C_3$-$C_7$-Cycloalkyl oder gegebenenfalls durch $C_1$-$C_{12}$-Alkyl, $C_1$-$C_{12}$-Alkoxy, Halogen, Cyan oder $CF_3$ substituiertes Phenyl, steht.

Besonders bevorzugt sind die Verbindungen der allgemeinen Formel I', worin R' für Methyl oder Vinyl steht (Verbindungen der Formeln I'a und I'b).

Gegenstand der Erfindung ist auch die Verwendung der Verbindungen der allgemeinen Formel I oder der allgemeinen Formel I', einschließlich der Verbindungen der Formeln I'a und I'b als Monomerkomponenten in polymeren Systemen.

Die Verbindungen der allgemeinen Formel I bzw. I' sind aber auch für andere Anwendungen brauchbar, so stellen die Verbindungen wertvolle Synthesebausteine für chemische Synthesen dar, beispielsweise für Verbindungen mit pharmakologischer oder pestizider Wirksamkeit.

Als Beispiele für den Rest R in der allgemeinen Formel I bzw. den Rest R' in der allgemeinen Formel I' sind zu nennen Methyl, Ethyl, n-Propyl, Isopropyl, n-Butyl, sec.-Butyl, Isobutyl, Pentyl, Hexyl, Heptyl, Octyl, Vinyl, Allyl, Butenyl, Pentenyl, Hexenyl, Cyclopropyl, Cyclobutyl, Cyclopentyl, Cyclohexyl, Cycloheptyl und Phenyl. Das Phenyl kann, wie zuvor definiert, mono- oder di- oder polysubstituiert sein.

Ganz allgemein können die beim erfindungsgemäßen Verfahren eingesetzten Acyloine und 1,2-Diketone auch nach anderen Arbeitsweisen erhalten werden. So ist die Umsetzung von auch gegebenenfalls substituierten Norbornencarbaldehyden mit dem Aldehyd der allgemeinen Formel II, R-CH = O, auch durch Übertragung der Maßnahmen in Synthesis 1977, 403, von Interesse, wobei eine Trennung der gebildeten unsymmetrischen Acyloine von den gleichzeitig gebildeten symmetrischen Acyloinen für Verbindungen mit R = $CH_3$ bis $C_4H_9$ destillativ und bei höheren Homologen und Derivaten thermolytisch erfolgen kann. Allerdings sind hierbei sterische Grenzen gesetzt. Setzt man nämlich 3-Methyl-norborn-5-en-2-carbaldehyd mit Propionaldehyd unter 1,3-Thiazoliumsalzkatalyse um, so erhält man eine Ausbeute an unsymmetrischem Acyloin von nur 29 % der Theorie. Bietet man dem Aldehyd nur sich selbst als Reaktionspartner an, erhält man nach dieser Arbeitsweise überhaupt kein Acyloin. Die Methylgruppe am Kohlenstoffatom 3 verhindert wirksam jede nukleophile Addition des Katalysators an die C = O-Gruppe des Aldehyds, vgl. Synthesis 1980, 1, 309.

Ein weiteres Verfahren ist in Chem. Ber. 112, 2062 (1978) und Chem. Ber. 114, 959 (1981) beschrieben. In Betracht kommt auch die in Synthesis 1969, 17, vorgeschlagene Arbeitsweise, bei der jedoch die Hydrolyse des intermediär gebildeten Thioketals nicht unproblematisch ist, vgl. auch Synthesis 1977, 357.

Zusammenfassend ist aber festzuhalten, daß die Herstellung der Acyloine und 1,2-Diketone aus der Kenntnis an sich bekannter Arbeitsweisen dem Fachmann möglich ist, so daß sich bei Anwendung der Pyrolysestufe des erfindungsgemäßen Verfahrens entweder in Verbindung mit den anderen Maßnahmen der Verfahrensansprüche oder der obigen Maßnahmen des genannten Standes der Technik sämtliche Vinylglyoxalderivate der allgemeinen Formeln I und I' herstellen lassen.

Das erfindungsgemäße Verfahren wird nachstehend insbesondere unter Bezugnahme auf die bevorzugten Verbindungen der Formeln I'a und I'b näher erläutert.

Die Synthese der Verbindungen I'a und I'b erfolgt nach dem erfindungsgemäßen Verfahren durch Gasphasenpyrolyse der Diketone III bzw. IV bei Temperaturen von 500 - 700° C, vorzugsweise 600° C und vermindertem Druck, insbesondere bei Drücken $<10^{-1}$ mbar, vorzugsweise bei ca. $10^{-2}$ mbar.

Reaktionsschema:

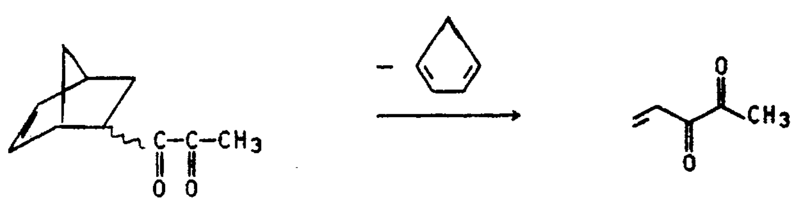

III I'a

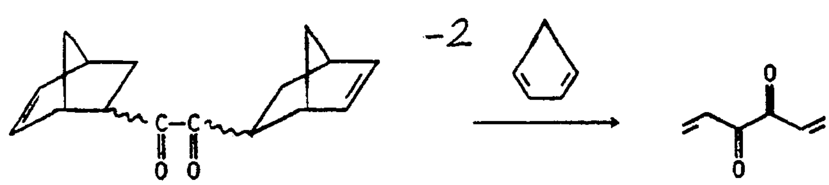


IV I'b

Als Edukte III und IV können die reinen exo- bzw. endo-Isomeren oder vorteilhafte die üblicherweise bei der Herstellung von III bzw. IV anfallenden exo/endo-Gemische eingesetzt werden.

Besonders zweckmäßig erfolgt die Pyrolyse beim erfindungsgemäßen Verfahren als sogenannte "Flash-Pyrolyse", da hierbei selbst thermolabile Produkte bei guten Ausbeuten gefaßt werden können.

Die Diketone der allgemeinen Formel I und I' sowie das bei der Pyrolyse gebildete Cyclopentadien

werden in Kühlfallen aufgefangen; die Diketone I und I' können ohne weitere Reinigung weiterverwendet werden.

Die als Edukte eingesetzten Diketone III und IV sind bekannt oder können nach an sich bekannten Verfahren synthetisiert werden. Aus Acrolein und Cyclopentadien erhält man nach Alder und Stein [Liebigs Ann. Chem. 525, 17 (1936)] Bicyclo-[2,2,1]-hept-5-en-carbaldehyd-1 in Form eines Gemisches der endo/exo-Isomeren (Verhältnis ca. 3:1).

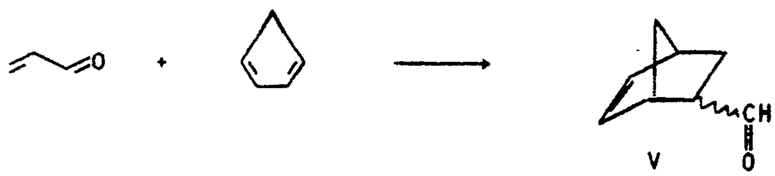

V

Eine Trennung des Isomerengemisches ist grundsätzlich möglich, aber im Hinblick auf den Pyrolyseschritt III → I'a bzw. IV → I'b nicht erforderlich. V wird anschließend nach der von Stetter et.al. vorgeschlagenen Methode (Syntheses 1976, 733; 1977, 1403, 1980, 1, 309) mit V bzw. Acetaldehyd (VI) zu den Acyloinen VII bzw. VIIIa, b umgesetzt.

Reaktionsschema:

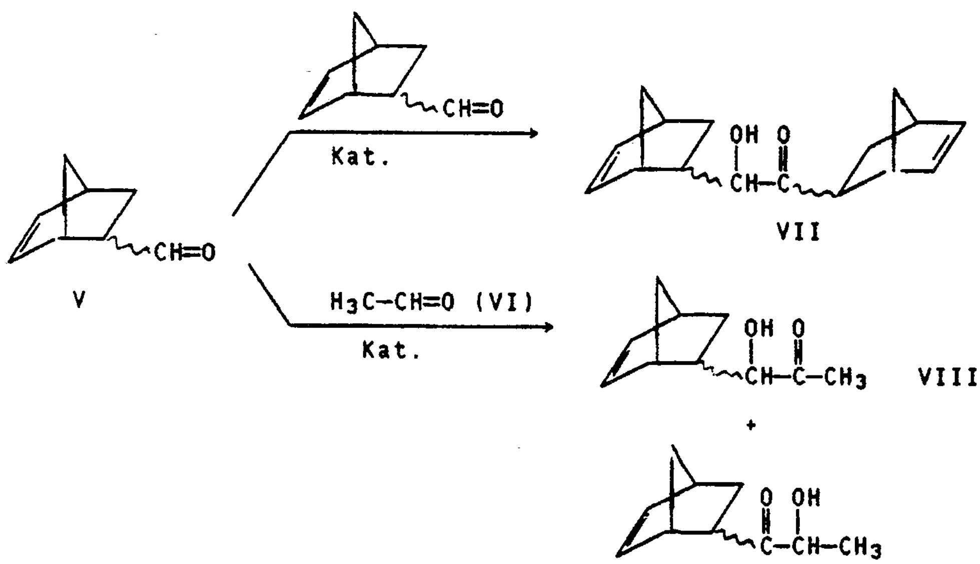


V

VII

VIII (a,b)

Als Katalysatoren kommen die für Acyloinkondensationen üblicherweise verwendeten in Frage, wie sie z.B. aus Houben-Weyl, Methoden der organischen Chemie, 4. Auflage, Band 7/2c, S. 2207/2207 bekannt sind und worauf hiermit Bezug genommen wird. Bevorzugt wird 3-Benzyl-5-(2-hydroxyethyl)-4-methyl-1,3-thiazoliumchlorid [Chem. Ber. 109, 2890 (1976), Synthesis 1975, 379] verwendet.

Die Oxidation der Acyloine VII bzw. VIII a,b zu den Diketonen III (a,b) bzw. IV kann nach bekannten Methoden (Houben-Weyl, Methoden der organischen Chemie, 4. Auflage, Band 7/2c, S 2233, worauf hiermit Bezug genommen wird) erfolgen, vorteilhaft wird $Bi_2O_3$ in Eisessig als Oxidationsmittel (J. chem. Soc. 1950, 2744 und 1951, 793) eingesetzt. Das bei der Reaktion aus $Bi_2O_3$ entstandene elementare Wismut kan durch Filtration leicht vom Reaktionsansatz abgetrennt und z.B. durch Erhitzen an der Luft wieder in $Bi_2O_3$ übergeführt werden.

Die Erfindung wird durch die nachstehenden Beispiele weiter erläutert.

Beispiel 1

a) Herstellung von Endo- und exo-Bicyclo-(2,2,1)-hept-5-en-2-carbaldehyd (V)

Zu einer Lösung von 66,0 g (1,00 mol) frisch gespaltetem Cyclopentadien in 50 ml absolutem Diethylether tropft man 60,0 g (1,10 mol) frisch destilliertes Acrolein binnen 10 Minuten zu. Während des

Zutropfens wird mit Eiswasser gekühlt. Nach einiger Zeit ist ein gelindes Sieden des Ethers zu erkennen, das aber bald wieder zum Stillstand kommt. Dann entfernt man die Kühlung und erhitzt noch eine halbe Stunde unter Rückfluß. Das erkaltete Reaktionsgemisch wird mit 200 ml Ether versetzt, mehrmals mit Wasser gewaschen und getrocknet. Das Lösungsmittel wird abgezogen und der Rückstand fraktioniert destilliert.

$Sdp._{15}$: 61 - 65 °C

Ausbeute: 107 g (0,88 mol, 88 %)

b) Herstellung des Katalysators 3-Benzyl-5-(2-hydroxyethyl)-4-methyl-1,3-thiazoliumchlorid (X)

In einem 500 ml Rundkolben werden 71,6 g (500 mmol) 5-(2-Hydroxyethyl)-4-methyl-1,3-thiazol), 63,3 g (500 mmol) frisch destilliertes Benzylchlorid und 250 ml absolutes Acetonitril 24 h unter Rückfluß erhitzt. Das Reaktionsgemisch wird unter Rühren langsam auf Raumtemperatur abgekühlt, der Niederschlag abgesaugt und mit trockenem Acetonitril farblos gewaschen, vorgetrocknet und dann im Wasserstrahlvakuum bei 90 °C getrocknet.

Schmp.: 139 - 140 °C

Ausbeute: 108,8 g (400 mmol, 80 %)

c) Herstellung von 1,2-Bis-[bicyclo-(2,2,1)-hept-5-en-2-yl]-ethan-1-on-2-ol (VII)

In einem 250 ml Dreihalskolben mit Rührer, Rückflußkühler (mit Trockenrohr) und mit Gasaufleitungsrohr für trockenen Stickstoff werden 100,0 g (818 mmol) V, 6,7 g (25 mmol) X, 150 ml absolutes Ethanol und zuletzt 15,2 g (150 mmol) trockenes Triethylamin gegeben und 12 h im schwachen Stickstoffstrom erhitzt. Die Reaktion wird dabei gaschromatographisch verfolgt. (Säulenmaterial: OV 17 auf Chromosorb® WAW 80/100, 48 cm; 10 % UCC W 982 auf Chromosorb WAW DMCS, 48 cm). Nach dem Erkalten wird das Reaktionsgemisch auf einen halben Liter Eis/Wasser gegeben und mit 300 ml Chloroform extrahiert. Die organische Phase wird mit verdünnter Schwefelsäure, Wasser und Natriumhydrogencarbonat-Lösung gewaschen und getrocknet. Chloroform wird anschließend abgezogen und der Rückstand fraktioniert destilliert.

$Sdp._{0,01}$: 128 - 131 °C

Schmp.: 52 - 55 °C

Ausbeute: 65,1 g (266 mmol, 65 %)

d) Herstellung von 1-[Bicyclo-(2,2,1)-hept-5-en-2-yl]-propan-1-ol-2-on (VIII)

In einem Autoklaven (100 ml) mit Magnetrührer werden 12,2 g (100 mmol) V, 40 ml (ca. 0,7 mol) Acetaldehyd, 2,70 g (10,0 mmol) und 15 ml absolutes Triethylamin durch ein heißes Ölbad (90 °C) 5 h erhitzt. Nach dem Erkalten wird das Reaktionsgemisch auf 200 ml Eis/Wasser gegossen. Zehn Autoklavenfüllungen (es stand kein größeres Gerät zur Verfügung) werden gesammelt und mit 500 ml Chloroform extrahiert. Die organische Phase wird mit verdünnter Schwefelsäure, Wasser und Natriumhydrogencarbonatlösung gewaschen und über Magnesiumsulfat getrocknet. Das Chloroform wird abgezogen und der Rückstand fraktioniert destilliert.

$Sdp.:_{0,01}$: 47 - 49 °C

Ausbeute: 74,8 g (450 mmol, 45 % bezogen auf V)

e) Herstellung von 1,2-Bis-[bicyclo-(2,2,1)-hept-5-en-2-yl]-ethandion-(1,2) (IV)

49,9 g (204 mmol) VII werden in 130 ml 2-Ethoxyethanol und 40 ml Eisessig unter Rühren auf 105 °C erhitzt. Dann werden auf einmal 25,0 g (53,6 mmol) $Bi_2O_3$ hinzugesetzt. Nach einer Reaktionszeit von zwei Stunden wird das elementare Wismut abfiltriert und mit 300 ml Chloroform gewaschen. Die organische Phase wird mit Wasser neutral gewaschen. Das Lösungsmittel wird abgezogen und der Rückstand fraktioniert destilliert. Anschließend wird aus Petrolether umkristallisiert.

$Sdp._{0,01}$: 98 - 100 °C

Schmp: 47 - 49 °C

Ausbeute: 32,0 g (132 mmol, 66 %)

Die Darstellung von III (endo- und exo-[Bicyclo-(2,2,1)-hept-5-en-2-yl-propan-1,2-dion] erfolgt analog.

$Sdp._{20}$: 94 - 95 °C

Ausbeute: 21,0 g (128 mmol, 64 %)

| | | | | |
|---|---|---|---|---|
| Elementaranalyse: $C_{10}H_{12}O_2$ (164,20) | ber.: | C 73,14 | H 7,37 |
| | gef.: | C 73,00 | H 7,37 |
| | | C 73,16 | H 7,41 |

f) Herstellung von Methylvinylglyoxal (I'a)

In einer Gasphasenpyrolyseapparatur, bestehend aus einem Kleinrohrofen mit Quarzrohr und einer damit verbundenen Vakuumlinie mit vier kühlfallen wird die Pyrolyse bei 600 °C und einem Druck von 0,01 bis 0,02 Torr durchgeführt. Die dem Ofen unmittelbar benachbarten Kühlfallen, in denen sich die gebildeten Produkte sammeln, werden auf -78 °C (Kühlfalle 1) und -178 °C (Kühlfalle 2) gekühlt. Zur Durchführung der Pyrolyse wird das Vorratsgefäß auf 60 °C aufgeheizt. Nach ca. 2 h ist das Edukt VIII aus dem Vorratsgefäß verschwunden, in der 1. Kühlfalle befindet sich das Dion I'a, in der 2. Kühlfalle das gleichzeitig gebildete Cyclopentadien. Verwendet man statt zwei Produktkühlfallen deren vier, so kühlt man die erste, vom Ofen aus gerechnet, auf -78 °C und die vierte auf -178 °C. Die beiden mittleren bleiben zunächst ungekühlt. Cyclopentadien tritt daher ohne zu kondensieren durch sie hindurch. Man hat auf diese Weise die Möglichkeit das Dion durch Umkondensation zu reinigen. Ausbeute an I'a: 89 %

$^1$H-NMR (80 MHz, $CDCl_3$]: 6.0-7.3 (ABC-m; 3 H, Vinyl-H), 2.47 (s; 3 H, $CH_3$).
- $^{13}$C-NMR ($CDCl_3$): 24,2 (q; $CH_3$), 128.7 (d; C-4), 133.1 (t; C-5), 186.8 (s; C-3), 197.9 (s; C-2).
- IR (Film): 1715 (C=O), 1690 (C=O) cm$^{-1}$.
- UV (Ethanol): $\lambda_{max}$ = 434 nm, $\epsilon$ = 18.

Beispiel 2

Divinylglyoxal (I'b)

Man verfährt analog Beispiel 1, Stufe f), heizt aber das Vorratsgefäß auf 110 °C auf. Ausbeute an I'b. 81 %.

$^1$H-NMR (80MHz, $CDCl_3$): 6,0 - 7,2 (ABC-m; 6 H, Vinyl-H).
$^{13}$C-NMR ($CDCl_3$): 130,1 (d; C-2, C-5), 133,0 (t; C-1, C-6), 189,0 (s; C-3, C-4).
IR (Film): 1690 cm$^{-1}$ (C=O).
UV (Ethanol): $\lambda_{max}$= 432, $\epsilon$ = 17
2: $^1$H-NMR (80 MHz, $CDCl_3$): 6,0 - 7,3 (ABC-m; 3 H, Vinyl-H), 2,47 (s; 3 H, $CH_3$).
$^{13}$C-NMR ($CDCl_3$): 24,2 g (q; $CH_3$), 128,7 (d; C-4), 133,1 (t; C-5), 186,8 (s; C-3), 197,9 (s; C-2).
IR (Film): 1715 (C=O), 1690 (C=O) cm$^{-1}$.
UV (Ethanol): $\lambda_{max}$= 434 nm, $\epsilon$ = 18.

I'b wurde mittels Pd/C zum 3,4-Hexadion hydriert, welches mit authentischem Material verglichen wurde und mit ihm identisch war.

Beispiele 3 bis 6

Man verfährt wie in Beispiel 1 (Stufen d bis f) angegeben, wobei man in Stufe d anstelle von Acetaldehyd folgende Aldehyde der Formel II einsetzt:
Beispiel 3) R = $C_2H_5$
4) R = n-$C_3C_7$
5) R = i-$C_3H_7$
6) R = $C_6H_5$

Die erhaltenen Acyloine der Formeln VIII werden mit $Bi_2O_3$ analog Beispiel 1e zu den entsprechenden Diketonen oxidiert, die der Gasphasenthermolyse (600 °C/0,01 Torr) entsprechend Beispiel 1f zu den Vinylglyoxalen der Formel I unterworfen werden (Ausbeute der Pyrolysestufe: in Klammer in % d.Th.):

Beispiel 3 R = $C_2H_5$ (80 %)

1-Hexen-3,4-dion

$^1$H-NMR (300 MHz, $CDCl_3$/TMS): $\delta$ (ppm) = 6.52 (dd, 1H, J = 18 Hz, $^2$J - 1.5 Hz); 6.05 (dd, 1H, J = 11 Hz, $^2$J = 1.5 Hz); 7.01 (dd, 1H, J - 18 Hz, J = 11 Hz); 2.84 (q, 2H, J = 7 Hz); 1.12 (t, 3H, J = 7 Hz)
$^{13}$C-NMR (75 MHz, $CDCl_3$/TMS): $\delta$ (ppm) = 133.5 (C-1); 129.3 (C-2); 187.9 (C-3); 201.2 (C-4); 30.2 (C-5); 6.9 (C-6)
IR-Sprektrum (Film, cm$^{-1}$): 2990, 2960, 2890, 1690, 1610, 1455, 1080, 980, 905
MS (70 eV, m/e): 112 ($M^+$, 10 %); 57 (100 %); 55 (73 %)
UV (Cyclohexan): $\lambda_{max}$ = 440 nm ($\epsilon$ = 21); 238 nm ($\epsilon$ = 5500)

Beispiel 4 R = n-$C_3H_7$ (80 %)

1-Hepten-3,4-dion

$^1$H-NMR (300 MHz, $CDCl_3$/TMS): $\delta$ (ppm) = 6.51 (dd, 1H, J = 17 Hz, $^2$J = 1.5 Hz); 6.05 (dd, 1H, J = 11 Hz, $^2$J = 1.5 Hz); 7.00 (dd, 1H, J = 17 Hz; J = 11 Hz); 2.79 (t, 2H, J = 7 Hz); 1.65 (tq, 2H, J = 7 Hz, J = 7 Hz); 0.96 (t, 3H, J = 7Hz)
$^{13}$C-NMR (75 MHz, $CDCl_3$/TMS): $\delta$ (ppm) = 133.4 (C-1); 129.3 (C-2); 187.9 (C-3); 200.7 (C-4); 38.6 (C-5); 16.6, 13.7 (C-6 oder C-7)
IR-Spektrum (Film, cm$^{-1}$): 2970, 2940, 1710, 1690, 1605, 1450, 1400, 1100, 950
MS (70 eV, m/e): 126 ($M^+$, 6 %); 61 (67 %); 55 (86 %); 43 (100 %)
UV Cyclohexan): $\lambda_{max}$ = 444 nm ($\epsilon$ = 21); 238 nm ($\epsilon$ = 5000)

Beispiel 5 R = iso-$C_3H_7$ (80 %)

5-Methyl-1-hexen-3,4-dion

$^1$H-NMR (300 MHz, $CDCl_3$/TMS): $\delta$ (ppm) = 6.47 (dd, 1H, J = 17 Hz, $^2$J = 1.5 Hz); 6.07 (dd, 1H, J = 11 Hz, $^2$J = 1.5 Hz); 6.93 (dd, 1H, J = 17 Hz, J = 11 Hz); 3.39 (sept., 1H, J = 7 Hz); 1.13 (d, 6H, J = 7Hz
$^{13}$C-NMR (75 MHz, $CDCl_3$/TMS): $\delta$ (ppm) = 133.5 (C-1); 130.3 (C-2); 189.3 (C-3); 204.2 (C-4); 34.6 (C-5); 17.2 (C-6, C-7)
IR-Spektrum (Film cm$^{-1}$): 2980, 2940, 2880, 1700, 1605, 1460, 1400, 975, 930, 845
UV (Cyclohexan): $\lambda_{max}$ = 449 nm ($\epsilon$ 22); 238 nm ($\epsilon$ = 400)
MS (70 eV, m/e): 126 ($M^+$, 5 %); 61 (41 %); 55 (64 %); 43 (100 %)

Beispiel 6 R = $C_6H_5$ (75 %)

1-Phenyl-3-buten-1,2-dion

$^1$H-NMR (300 MHz, $CDCl_3$/TMS): $\delta$ (ppm) = 6.41 (dd. 1H, J = 18 Hz, $^2$J = 0.6 Hz); 6.24 (dd, 1H, J = 11 Hz, $^2$J = 0.6 Hz); j6.75 (dd, 1H, J = 18 Hz, J = 11 Hz); 7.95-7.99 (m, $2H_{ortho}$); 7.47-7.54 (m, $2H_{meta}$); 7.62-7.68 (m, $1H_{para}$)
$^{13}$C-NMR (75 MHz, $CDCl_3$/TMS): $\delta$ (ppm) = 134.8 (C-4); 132.7 (C-3); 192.9, 193.3 (C-1, C-2); 132.5 ($C_i$); 130.0 ($2C_{ortho}$); 128.9 ($2c_{meta}$); 134.8 ($C_{para}$)

**Patentansprüche**

**1.** Verfahren zur Herstellung von Vinylglyoxalderivaten der allgemeinen Formel I

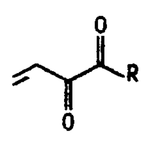

(I)

worin R für einen geradkettigen oder verzweigten Alkyl- oder Alkenylrest oder Cycloalkylrest oder einen gegebenenfalls substituierten Phenylrest steht, dadurch gekennzeichnet, daß man Bicyclo-[2,2,1]-hept-5-en-carbaldehyd der Formel V

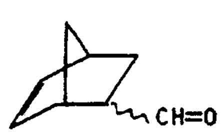

(V)

in Gegenwart eines Katalysators entweder

(a) mit sich selbst unter Bildung einer Verbindung der allgemeinen Formel VII

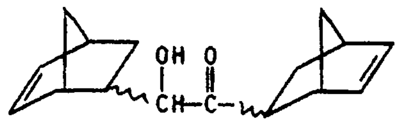

(VII)

reagieren läßt oder
(b) mit einer Verbindung der allgemeinen Formel II

R—CH=O (II)

worin R die oben angegebenen Bedeutungen besitzt, unter Bildung eines Gemisches der Verbindungen der allgemeinen Formeln VIII (a) und (b)

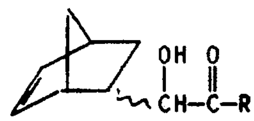

(VIII a)

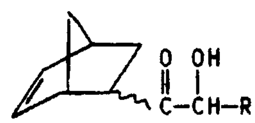

(VIII b)

worin R wie zuvor definiert ist, umsetzt,
die Verbindungen der allgemeinen Formeln VII beziehungsweise VIII (a) und (b) zu den entsprechenden Dicarbonylverbindungen oxidiert und anschließend bei einer Temperatur von 500 bis 700 °C und unter vermindertem Druck, insbesondere einem Druck von weniger als $10^{-1}$ mbar eine Gasphasenpyrolyse durchführt, und gewünschtenfalls die gebildeten Verbindungen der allgemeinen Formel I weiter reinigt.

2. Verfahren nach Anspruch 1, dadurch gekennzeichnet, daß man die Gasphasenpyrolyse bei 550 bis 630 °C und einem Druck von ungefähr $10^{-2}$ mbar durchführt.

3. Verfahren nach einem der Ansprüche 1 oder 2, dadurch gekennzeichnet, daß man als Katalysator 3-Benzyl-5-(2-hydroxyethyl)-4-methyl-1,3-thiazoliumchlorid einsetzt.

4. Verfahren nach einem der Ansprüche 1 bis 3, dadurch gekennzeichnet, daß man eine Verbindung der allgemeinen Formel II

R—CH=O (II)

einsetzt, worin R für $C_1$-$C_{20}$-Alkyl, insbesondere $C_1$-$C_4$-Alkyl, $C_2$-$C_{20}$-Alkenyl, insbesondere $C_2$-$C_6$-Alkenyl, $C_3$-$C_7$-Cycloalkyl, oder gegebenenfalls durch Halogen, Cyan, $C_1$-$C_{12}$-Alkyl, insbesondere $C_1$-$C_4$-Alkyl, $C_1$-$C_{12}$-Alkoxy, insbesondere $C_1$-$C_4$-Alkoxy, oder $CF_3$ substituiertes Phenyl, steht.

5. Verbindungen der allgemeinen Formel I'

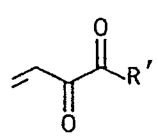

(I')

worin R' für Methyl, Ethyl, n-Propyl, iso-Propyl, n-Butyl, iso-Butyl, Hexyl, Heptyl, Octyl, $C_2$-$C_{20}$-Alkenyl, $C_3$-$C_7$-Cycloalkyl oder gegebenenfalls durch $C_1$-$C_{12}$-Alkyl, $C_1$-$C_{12}$-Alkoxy, Halogen, Cyan oder $CF_3$ substituiertes Phenyl, steht.

6. Verbindungen der allgemeinen Formel I' gemäß Anspruch 5, worin R' für Methyl, Ethyl, n-Propyl, iso-Propyl, n-Butyl, iso-Butyl, $C_2$-$C_6$-Alkenyl, $C_3$-$C_7$-Cycloalkyl oder gegebenenfalls durch $C_1$-$C_4$-Alkyl, $C_1$-$C_4$-Alkoxy, Halogen, Cyan oder $CF_3$ substituiertes Phenyl, steht.

7. Verbindungen der allgemeinen Formel I', worin R' für Methyl oder Vinyl steht.

8. Verwendung der Verbindungen der allgemeinen Formel I, gemäß Anspruch 1 oder der allgemeinen Formel I' gemäß einem der Ansprüche 5 bis 7 als Monomerkomponenten in polymeren Systemen.

## Claims

1. A process for the preparation of a vinylglyoxal derivative of the general formula I

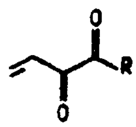

(I)

where R is straight-chain or branched alkyl or alkenyl, cycloalkyl or unsubstituted or substituted phenyl, wherein bicyclo[2.2.1]hept-5-enecarbaldehyde of the formula V

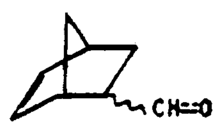

V

is either

(a) subjected to autocondensation with formation of a compound of the formula VII

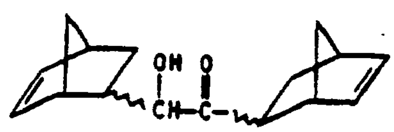

(VII)

or

(b) reacted with a compound of the general formula II

R-CH = O (II)

where R has the above meanings, with formation of a mixture of the compounds of the general formulae VIII (a) and (b)

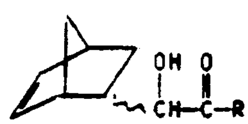

(VIII a)

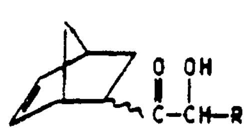

(VIII b)

where R is as defined above, in the presence of a catalyst, and the compound of the formula VII or the compounds of the formulae VIII (a) and (b) is or are oxidized to the corresponding dicarbonyl compounds, after which gas-phase pyrolysis is carried out at from 500 to 700 °C and under reduced pressure,,in particular less than $10^{-1}$ mbar, and, if desired, the resulting compound of the general formula I is further purified.

2. A process as claimed in claim 1, wherein the gas-phase pyrolysis is carried out at from 550 to 630 °C and under about $10^{-2}$ mbar.

3. A process as claimed in claim 1 or 2, wherein the catalyst used is 3-benzyl-5-(2-hydroxyethyl)-4-methyl-1,3-thiazolium chloride.

4. A process as claimed in one of claims 1 to 3, wherein a compound of the general formula II

R-CH=O (II)

is used, where R is $C_1$-$C_{20}$-alkyl, in particular $C_1$-$C_4$-alkyl, $C_2$-$C_{20}$-alkenyl, in particular $C_2$-$C_6$-alkenyl, $C_3$-$C_7$-cycloalkyl, or phenyl which is unsubstituted or substituted by halogen, cyano, $C_1$-$C_{12}$-alkyl, in particular $C_1$-$C_4$-alkyl, $C_1$-$C_{12}$-alkoxy, in particular $C_1$-$C_4$-alkoxy, or $CF_3$.

5. A compound of the general formula I'

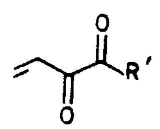

(I')

where R' is methyl, ethyl, n-propyl, isopropyl, n-butyl, isobutyl, hexyl, heptyl, octyl, $C_2$-$C_{20}$-alkenyl, $C_3$-$C_7$-cycloalkyl, or phenyl which is unsubstituted or substituted by $C_1$-$C_{12}$-alkyl, $C_1$-$C_{12}$-alkoxy, halogen, cyano or $CF_3$.

6. A compound of the formula I' as claimed in claim 5, where R' is methyl, ethyl, n-propyl, isopropyl, n-butyl, isobutyl, $C_2$-$C_6$-alkenyl, $C_3$-$C_7$-cycloalkyl, or phenyl which is unsubstituted or substituted by $C_1$-$C_4$-alkyl, $C_1$-$C_4$-alkoxy, halogen, cyano or $CF_3$.

7. A compound of the general formula I', where R' is methyl or vinyl.

8. The use of a compound of the formula I as claimed in claim 1 or of the formula I' as claimed in one of claims 5 to 7 as a monomer component in a polymeric system.

**Revendications**

1. Procédé de préparation de dérivés de vinylglyoxal de formule générale I

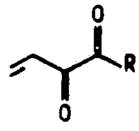

(I)

dans laquelle R est mis pour un reste alkyle ou alcényle a chaîne droite ou ramifiée ou pour un reste cycloalkyle ou pour un reste phényle éventuellement substitué, caractérisé en ce que l'on fait réagir le bicyclo-[2,2,1]-hept-5-ène-carbaldéhyde de formule V

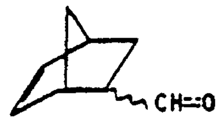

(V)

en présence d'un catalyseur,

(a) soit avec lui-même, ce qui engendre la formation d'un composé de formule générale VII

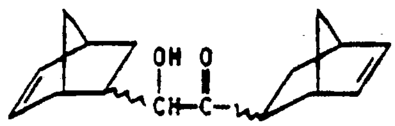

(VII)

(b) soit avec un composé de formule générale II

R-CH=O (II)

dans laquelle R a les significations sus-indiquées, ce qui entraîne la formation d'un mélange des composés des formules générales VIII (a) et VIII (b)

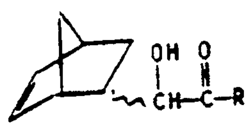

(VIII a)

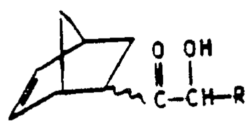

(VIII b)

dans lesquelles R est tel que défini ci-dessus,

on oxyde les composés des formules générales VII ou bien VIII (a) et VIII (b) en les composés dicarbonylés correspondants et, ensuite, on effectue une pyrolyse en phase gazeuse à une température de 500 à 700° C et sous pression reduite, en particulier une pression inférieure à $10^{-1}$ mbar, et, si on le désire, on purifie davantage les composés formés de la formule générale I.

**2.** Procédé suivant la revendication 1, caractérisé en ce que l'on effectue la pyrolyse en phase gazeuse à une température de 550 à 630° C et sous une pression d'environ $10^{-2}$ mbar.

**3.** Procédé suivant l'une des revendications 1 ou 2, caractérisé en ce que l'on utilise, comme catalyseur, le chlorure de 3-benzyl-5-(2-hydroxyethvl)-4-méthyl-1,3-thiazolium.

**4.** Procédé suivant l'une des revendications 1 à 3, caractérisé en ce que l'on utilise un composé de la formule générale II

R-CH=O (II)

dans laquelle R est mis pour un radical $C_1$-$C_{20}$-alkyle,en particulier $C_1$-$C_4$-alkyle, un radical $C_2$-$C_{20}$-alcényle, en particulier $C_2$-$C_6$-alcényle, un radical $C_3$-$C_7$-cycloalkyle, ou un radical phényle éventuellement substitué par des atomes d'halogène, des restes cyano, $C_1$-$C_{12}$-alkyle, en particulier $C_1$-$C_4$-alkyle, $C_1$-$C_{12}$-alcoxy, en particulier $C_1$-$C_4$-alcoxy, ou $CF_3$.

**5.** Composés de formule générale I'

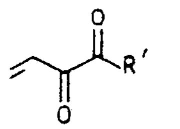

(I')

dans laquelle R' est mis pour un radical méthyle, éthyle, n-propyle, isopropyle, n-butyle, isobutyle, hexyle, heptyle, octyle, $C_2$-$C_{20}$-alcényle, $C_3$-$C_7$-cycloalkyle ou phényle éventuellement substitué par un reste $C_1$-$C_{12}$-alkyle, $C_1$-$C_{12}$-alcoxy, halogéno, cyano ou $CF_3$.

**6.** Composés de la formule générale I' suivant la revendication 5, formule dans laquelle R' est mis pour un radical methyle, éthyle, n-propyle, isopropyle, n-butyle, isobutyle, $C_2$-$C_6$-alcényle, $C_3$-$C_7$-cycloalkyle ou phényle éventuellement substitué par un reste $C_1$-$C_4$-alkyle, $C_1$-$C_4$-alcoxy, halogéno, cyano ou $CF_3$.

**7.** Composés de la formule générale I', formule dans laquelle R' est mis pour un radical methyle ou vinyle.

**8.** Utilisation des composés de la formule générale I, suivant la revendication 1, ou de la formule générale I' suivant l'une des revendications 5 à 7, comme composants monomères dans des systèmes polymères.